# EUROPEAN PATENT APPLICATION

(11) **EP 4 792 805 A1**
(43) Date of publication of application: **19.08.2026**
(21) Application number: 26158532.7
(22) Date of filing: 13.02.2026
(51) Int. Cl.: A61K 9/127, A61K 47/22, A61K 47/24, A61K 47/28, A61K 48/00

(54) **A BICELLE ENTITY, DNA ORIGAMI ENVELOPE, A BIOLOGICAL COMPLEX, HYBRID PORE DEVICE COMPRISING THE SAME**

(30) Priority: 14.02.2025 EP 25158032
(71) Applicant: Imec VZW, 3001 Leuven (BE); RIJKSUNIVERSITEIT GRONINGEN, 9712 CP Groningen (NL); Katholieke Universiteit Leuven, 3000 Leuven (BE)
(72) Inventor: GEVERS, Juliette, 2140 Borgerhout (BE); MARTENS, Koen, 9880 Aalter (BE); VAN DORPE, Pol, 3510 Spalbeek (BE); MAGLIA, Giovanni, 9747 AG Groningen (NL)
(74) Representative: Winger

(57) **Abstract**

The invention relates to a bicelle entity comprising a bicelle structure with a rim and first and second amphiphilic compounds, the first having a longer hydrophobic chain, and at least one extension configured to bind a DNA origami envelope or its extension. It further relates to a DNA origami envelope adapted for such binding and to a complex formed by their assembly. A kit comprising the bicelle entity and the DNA origami envelope is also provided. The invention additionally concerns a hybrid pore device incorporating the complex with a solid-state pore. Methods are disclosed for manufacturing the complex by assembling the bicelle entity and DNA origami envelope, and for producing the hybrid pore device by integrating the complex with the solid-state pore.

## Description

### Technical field

The invention is related to a biological complex, more specifically related to a biological complex comprising a bicelle structure. It is also related to a hybrid nanopore device comprising the same.

### Technical background

Bicelles are valuable tools in numerous research fields, including protein studies. They provide a stable and consistent environment, facilitating the investigation of membrane-associated biomolecules. As advanced models of biological membranes, bicelles enable detailed exploration of the structure, dynamics, and topology of membrane proteins. However, the use of bicelles requires additional stabilization.

### Summary of invention

The invention is set out in the appended set of claims.

Stabilizing bicelles within DNA origami structures is challenging due to the inherent differences in their structural and chemical properties. Bicelles, being lipid-based nanostructures, and DNA origami, which involves the precise folding of DNA strands into specific shapes, have fundamentally different properties. This disparity makes it difficult to integrate them without compromising the stability of either component.

It is an object of the present disclosure to provide a stable bicelle structure for and in a DNA origami structure, e.g. DNA origami envelope.

In the first aspect, the present invention relates to a bicelle entity comprising a bicelle structure and at least one extension. The bicelle structure comprises a first and a second amphiphilic compound wherein the first amphiphilic compound has longer hydrophobic chain length than the second amphiphilic compound. The at least one extension projects from the rim, wherein the at least one extension is configured for binding to a DNA origami envelope or an extension of the DNA origami envelope. In embodiments, the first amphiphilic compound forms the central bilayered disc and has longer hydrophobic chain length than the second amphiphilic compound, which forms the rim surrounding the central disc. The DNA origami envelope can provide a scaffold that further stabilizes the bicelle structure, preventing it from disassembling under various conditions.

In embodiments, the first and second amphiphilic compounds are phospholipids.

In embodiments, the bicelle structure is a lipid-based nanostructure comprising a mixture of the first long-chain and the second short-chain phospholipids. In embodiments, the bicelle structure is a lipid-based nanostructure comprising a mixture of the first and the second phospholipids wherein the first phospholipid has longer hydrophobic chain length than the second phospholipid.

In embodiments, the size of bicelles can be set by adjusting the first amphiphilic compound/second amphiphilic compound ratio. In embodiments, the first amphiphilic compound may have charged hydrophilic head groups, such as 1,2-stearoyl-3-trimethylammonium-propane. This allows manipulating the surface charge.

In embodiments, the at least one extension comprises a hydrophobic moiety and is incorporated into the bicelle structure via said hydrophobic moiety. Hydrophobic moieties can provide a bond between the bicelle structure and the at least one extension in the bicelle entity. In embodiments, the hydrophobic moiety is optionally selected from the list consisting of cholesterol, porphyrin, tocopherol, and a lipid-handle.

In embodiments, the extension comprises a functional moiety for binding to the DNA origami envelope or an extension of the DNA origami envelope.

In embodiments, the functional moiety is selected from the list consisting of a nucleic acid, an alkyne, an azide, a streptavidin, and a biotin moiety. In embodiments, the functional moiety is selected from a DNA oligo nucleic acid, an RNA oligo nucleic acid, a biotin, a streptavidin, an azide, or a dibenzocyclooctyne (DBCO) moiety. In embodiments, the functional moiety is a single strand DNA oligo nucleic acid, i.e., a ssDNA oligo nucleic acid. Complementary base pairing between ssDNAs ensures high specificity and stability of the final structure. Biotin as a functional moiety binds tightly and with high specificity to streptavidin. Alkyne functional moieties such as hexynyl or octadiynyl, can bind tightly and with high specificity to azide groups via the azide alkyne Huisgen cycloaddition click reaction. Alkyne functional moieties such as Dibenzocyclooctyne (DBCO) can also bind to azide groups, here via the copper-free click reaction.

In embodiments, the functional moiety can be a nucleic acid which attaches to the rim, or a chemical group or a molecule attached to the rim, which can bind to a complementary chemical group or molecule via specific interactions or reactions. The functional moiety of the bicelle entity can bind to a complementary functional moiety from the DNA origami envelope or to a complementary functional moiety from an extension of the DNA origami envelope.

In embodiments, a Critical Packing Parameter (CPP) of the first amphiphilic compound is in the range between 0.5 to 1.1, more preferably in the range between 0.9 to 1.1. In embodiments, the Critical Packing Parameter is substantially 1. When the CPP is around 1, the geometry of the first amphiphilic compound favors the formation of flat, bilayer structures, which is characteristic of the disk-like shape of bicelle structures.

In embodiments, the first amphiphilic compound is selected from 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), and 1-palmitoyl-2-oleoyl-glycero-3-phosphocholine (POPC).

In embodiments, the bicelle structure comprises at least a third amphiphilic compound having a Critical Packing Parameter in the range between 0.5 to 1.1, more preferably in the range between 0.9 to 1.1. Thus, the flat, central bilayer structure is formed by at least two different types of amphiphilic compounds. In embodiments the at least a third amphiphilic compound may either comprise a positively charged hydrophilic head group or may comprise a negatively charged head group. This permits to manipulate the surface charge of the bilayer disc. In embodiments, the third amphiphilic compound with a positively charged head group may be selected from 1,2-stearoyl-3-trimethylammonium-propane (DOTAP), 1,2-di-O-octadecenyl-3-trimethylammonium-propane (DOTMA), 1,2-dioleoyl-sn-glycero-3-phosphoethanolamine (DOPE). In embodiments, the third amphiphilic compound with a negatively charged head group may be selected from phosphatidylinositol phosphates (PIP), phosphatidylserine (PS), and phosphatidic acid (PA).

In embodiments, a Critical Packing Parameter (CPP) of the second amphiphilic compound is below or equal to 0.5. It is advantageous that the short-chain second amphiphilic compounds helps stabilize the edges of the bicelle, preventing the planar bilayer from curling into vesicles or other structures.

In embodiments, the second amphiphilic compound is selected from 1,2-dihexanoyl-sn-glycero-3-phosphocholine (06:00 DHPC), 1,2-diheptanoyl-sn-glycero-3-phosphocholine (07:00 DHPC), and 3-[(3-Cholamidopropyl)dimethylammonio]-2-hydroxy-1-propanesulfonate (CHAPSO).

In embodiments, the bicelle structure comprises a fourth amphiphilic compound having a Critical Packing Parameter below or equal to 0.5. Thus, the flat bilayer structure is stabilized by at least two different types of amphiphilic compound forming the rim.

In embodiments, the bicelle structure consists of a first and a second type of amphiphilic compounds. Thus, the flat bilayer structure is formed homogeneously by one type of long-chain amphiphilic compound and is stabilized homogeneously by one type of short-chain amphiphilic compound forming the rim. In other words, the flat bilayer structure is formed homogeneously by one type of longer-chain amphiphilic compound and is stabilized homogeneously by one type of shorter-chain amphiphilic compound forming the rim.

In embodiments, the bicelle entity is configured so that the rim can be surrounded by the DNA origami envelope. In embodiments, the position of at least one extension is near (e.g., at) the rim of the bicelle structure. Thus, when binding with the corresponding DNA origami envelope (e.g., via extensions of the DNA origami envelope), the bicelle entity is surrounded by the DNA origami envelope from a side view and/or a top view.

In embodiments, the at least one extension is at least two extensions and the bicelle entity is configured so that the rim of the bicelle structure can be entirely surrounded by the DNA origami envelope. In embodiments, a plurality of extensions are spread around the rim of the bicelle structure such that, during binding between the bicelle structure and the DNA origami envelope (e.g., via extensions of the DNA origami envelope), the bonds settle the bicelle structure inside the DNA origami envelope, thereby positioning the bicelle entity such that the rim of the bicelle structure is entirely surrounded by the DNA origami envelope. Thus, when binding with the corresponding DNA origami envelope (e.g., via extensions of the DNA origami envelope), the bicelle entity is surrounded by the DNA origami envelope from a side view and a top view.

In the second aspect, the invention relates to a DNA origami envelope configured for binding to the at least one extension of the bicelle entity described in the first aspect. This allows binding the bicelle entity to the DNA origami envelope. This advantageously allows the DNA origami envelope to further stabilize the bicelle entity.

In embodiments, the DNA origami envelope comprises at least one extension for binding to the at least one extension of the bicelle entity.

In embodiments, the DNA origami envelope comprises at least one extension for complementary binding to the at least one extension of the bicelle entity.

In embodiments, the DNA origami envelope comprises at least two extensions for binding to the at least two extensions of the bicelle entity.

In embodiments, the at least one extension of the DNA origami envelope comprises at least one functional moiety to bind to the corresponding functional moiety of the extension of the bicelle entity.

In embodiments, the functional moiety of the DNA origami envelope is selected from the list consisting of a nucleic acid, an alkyne, an azide, a biotin, and a streptavidin moiety. In embodiments, the functional moiety is selected from a DNA oligo nucleic acid, an RNA oligo nucleic acid, a biotin, a streptavidin, an alkyne, and an azide. In embodiments, the functional moiety is a single strand DNA oligo nucleic acid, i.e. ssDNA oligo nucleic acid.

In embodiments, the DNA origami envelope (20) may be configured for binding to the at least one extension (13) of the bicelle entity (10) by comprising at least one extension, such as a ssDNA oligo nucleic acid (21), extending inward from an inner surface of the DNA origami envelope (20).

In embodiments, the DNA origami envelope is configured for binding to the at least one extension of the bicelle entity by comprising at least one single strand DNA oligo nucleic acid, i.e., a ssDNA oligo nucleic acid, as the extension extending inward from an inner surface of the DNA origami envelope. The ssDNA oligo nucleic acid of the DNA origami envelope complements the ssDNA oligo nucleic of the bicelle entity. Thus, when binding the bicelle entity with the corresponding DNA origami envelope, the bicelle entity is surrounded by the DNA origami envelope from a side view and/or a top view.

In embodiments, the DNA origami envelope is configured for binding to the at least one extension of the bicelle entity by comprising at least one streptavidin, as the extension extending inward from an inner surface of the DNA origami envelope. The streptavidin of the DNA origami envelope complements the biotin functional moiety of the bicelle entity. Thus, when binding the bicelle entity with the corresponding DNA origami envelope, the bicelle entity is surrounded by the DNA origami envelope from a side view and/or a top view. In alternative embodiments, the DNA origami envelope may comprise at least one biotin and the bicelle entity may comprise at least one streptavidin.

In embodiments, the DNA origami envelope is configured for binding to the at least one extension of the bicelle entity by comprising at least one azide, as the extension extending inward from an inner surface of the DNA origami envelope. The azide of the DNA origami envelope complements the alkyne functional moiety of the bicelle entity. Thus, when binding the bicelle entity with the corresponding DNA origami envelope, the bicelle entity is surrounded by the DNA origami envelope from a side view and/or a top view. In alternative embodiments, the DNA origami envelope may comprise at least one alkyne functional moiety and the bicelle entity may comprise at least one azide.

In embodiments, the DNA origami envelope, wherein the DNA origami envelope is configured for binding to the at least one extension of the bicelle entity by comprising at least one azide, has the extension extending inward from an inner surface of the DNA origami envelope. The azide of the DNA origami envelope complements the DBCO functional moiety of the bicelle entity. Thus, when binding the bicelle entity with the corresponding DNA origami envelope, the bicelle entity is surrounded by the DNA origami envelope from a side view and/or a top view.

In embodiments, the DNA origami envelope is configured for binding to the at least one extension of the bicelle entity such that the rim of the bicelle structure can be entirely surrounded by the DNA origami envelope. Thus, when binding the bicelle entity with the corresponding DNA origami envelope, the bicelle entity is surrounded by the DNA origami envelope from a side view and a top view.

In embodiments, the DNA origami envelope can be in a predetermined shape, such as in a ring shape or a square shape.

In embodiments, the DNA origami envelope comprises a further dsDNA tail.

In the third aspect, the invention relates to a complex which comprises a bicelle structure binding to a DNA origami envelope or an extension of the DNA origami envelope.

In embodiments, the complex comprises at least one extension that binds the bicelle structure to the DNA origami envelope. The extension may correspond to the one described in the first aspect. So, in embodiments, the at least one extension is bonded to the bicelle structure via a hydrophobic moiety (e.g., that anchors it within the bicelle's rim), thereby forming a bicelle entity, and is also bound to the DNA origami envelope via a functional moiety of the extension (e.g., via a complementary moiety of an extension of the DNA origami envelope).

In embodiments, the complex comprises the bicelle entity described in the first aspect and the DNA origami envelope described in the second aspect.

In embodiments, the bicelle entity is bonded to the DNA origami envelope via a corresponding functional moiety of the at least one extension. In embodiments, the bicelle entity is bonded to the DNA origami envelope via a complementary functional moiety. In embodiments, the functional moiety is selected from the list consisting of nucleic acids and a biotin moiety. In embodiments, the functional moiety is selected from a DNA oligo nucleic acid, a RNA oligo nucleic acid, a biotin, an alkyne, and a dibenzocyclooctyne (DBCO). In embodiments, the functional moiety is a single strand DNA oligo nucleic acid, i.e., ssDNA oligo nucleic acid.

In embodiments, the bicelle entity and the DNA origami envelope may be bonded via complementary ssDNA oligo nucleic acids, complementary RNA oligo nucleic acid, an alkyne-azide click reaction, or a biotin streptavidin interaction.

In embodiments, the rim of the bicelle structure is surrounded by the DNA origami envelope.

In embodiments, the rim of the bicelle structure is entirely surrounded by the DNA origami envelope.

In embodiments, the complex further comprises a protein incorporated into the bicelle structure. In embodiments, the protein can be a membrane protein, such as a biological nanopore. The complex advantageously fixes the protein therein; thus the complex can be used for characterizing the protein in its specific native environment with, e.g., NMR (nuclear magnetic resonance spectroscopy) and EM (electron microscopy).

In embodiments, the protein in the complex is a biological pore. Thus, the bicelle entity in the complex can serve as an interposer for positioning the biological pore on a surface.

In the fourth aspect, the invention relates to a kit of parts comprising a bicelle entity according to the first aspect and a DNA origami envelope configured for binding to the at least one extension of the bicelle entity.

In the fifth aspect, the invention relates to a hybrid pore device comprising a complex according to the third aspect.

In embodiments, the biological pore in the complex and a solid-state pore are aligned to allow the passage of an analyte entity through both, the biological pore and the solid-state pore.

In embodiments, the hybrid pore comprises a substrate or membrane crossed by (or punctured by) the solid-state pore, wherein a top surface of the substrate surrounding an orifice of the solid-state pore comprises at least a hydrophobic area for assembling the complex to the top surface.

In embodiments, the substrate comprises Si, SiN, and/or SiOx.

In embodiments, the hybrid pore device further comprises a sensor configured to sense electrical or optical signals in the solid-state pore. In embodiments, the substrate comprises the sensor.

In embodiments, the substrate comprises an embedded conductive layer for sensing electrical signals in the solid-state pore.

In the sixth aspect, the invention relates to a method of manufacturing the complex in the third aspect. The method comprises steps of:
a. Providing a bicelle entity according to the first aspect;
b. Providing a DNA origami envelope according to the second aspect;
c. assembling the bicelle entity and the DNA origami envelope.

Assembling a bicelle structure and DNA origami, in general, remains challenging. Providing the bicelle entity with the extension and the DNA origami for assembling allows the connection between the hydrophobic bicelle entity and the hydrophilic DNA origami envelope. The binding between the at least one extension in the bicelle entity and the corresponding DNA origami envelope surprisingly integrates them into a stable complex.

In embodiments, step a and step b can be done in the order of step a then step b, or step b then step a, or step a and step b simultaneously. Step a and b can have time overlap during preparation. In embodiments, the bicelle entity and the DNA origami envelope can be pre-prepared and contained separately.

In embodiments, the concentration of the bicelle entity is above the critical micelle concentration (CMC) of the second amphiphilic compound, typically in the range of 1 to 100mM (milimolar).

In embodiments, the bicelle entity and the DNA origami envelope are provided in separate containers before step c of assembling the bicelle entity and the DNA origami envelope.

In embodiments, the bicelle entity and the DNA origami envelope are assembled such that the DNA origami envelope stabilizes the bicelle entity in step c of assembling the bicelle entity and the DNA origami envelope. The highly charged DNA origami envelope ensures that fusion of the bicelles is impossible by coulombic repulsion and steric hindrance, resulting in stabilization. This stabilization is even effective when the concentration of the bicelle entity is below the critical micelle concentration of the second amphiphilic compound. Conventionally, when the bicelle structure concentration is below the critical micelle concentration of the second amphiphilic compound, and without the provided stabilization by the DNA origami envelope, bicelle structures fuse together into large multilamellar structures.

In embodiments, step a of providing a bicelle entity according to the first aspect comprises a step a.1 of incubating the bicelle structure with an extension configured to incorporate into the DNA origami envelope, wherein said extension has a hydrophobic moiety. In embodiments, step a of providing a bicelle entity according to the first aspect comprises a step a.1 of incubating the bicelle structure with the extension configured for binding to the DNA origami envelope, wherein said extension has a hydrophobic moiety. Incubating the bicelle structure with the at least one extension beforehand ensures that the use of a detergent to stabilise the hydrophobic moiety in the DNA origami ring is not necessary. A detergent is unwanted because it cannot be removed selectively from the second amphiphilic compound.

In embodiments, when step a.1 is present, it may comprise a step a.11 of tuning the concentration of the bicelle entity (10) to be higher than the critical micelle concentration of the second amphiphilic compound.

In embodiments, the method further comprises a step x of tuning the concentration of the bicelle entity to be lower than the critical micelle concentration of the second amphiphilic compound. Step x is typically performed after step a.1 and before step c.

In embodiments, in step a, during a sub-step (step y) of forming the bicelle structure, the concentration of the second amphiphilic compound may be above the critical micelle concentration (CMC) of the second amphiphilic compound.

In embodiments, in step a, during a sub-step of incubating the bicelle structure with an extension configured to incorporate into the DNA origami envelope (or an extension of the DNA origami envelope), the concentration of the second amphiphilic compound may be above the critical micelle concentration (CMC) of the second amphiphilic compound.

In embodiments, after a sub-step (a.1) of incubating the bicelle structure with an extension configured to incorporate into the DNA origami envelope (or an extension of the DNA origami envelope), the concentration of the second amphiphilic compound may be brought (in a step x) below the critical micelle concentration (CMC) of the second amphiphilic compound, before performing step c. As a result, in the complex obtained after step c, the concentration of the second amphiphilic compound is below its critical micelle concentration (CMC).

In embodiments, in step a, during a sub-step (step y) of forming the bicelle structure, the concentration of the second amphiphilic compound is above the critical micelle concentration (CMC) of the second amphiphilic compound, then, during a subsequent step (a.1) of incubating the bicelle structure with an extension configured to incorporate into the DNA origami envelope (or an extension of the DNA origami envelope), the concentration of the second amphiphilic compound is above the critical micelle concentration (CMC) of the second amphiphilic compound, then,
after the sub-step (a.1) of incubating the bicelle structure with an extension configured to incorporate into the DNA origami envelope (or an extension of the DNA origami envelope), the concentration of the second amphiphilic compound is brought (in a step x) below the critical micelle concentration (CMC) of the second amphiphilic compound, before performing step c. As a result, in the complex obtained after step c, the concentration of the second amphiphilic compound is below its critical micelle concentration (CMC).

In embodiments, the step c of assembling the bicelle entity and the DNA origami envelope comprises adding the bicelle entity to the provided DNA origami envelope for assembling. In embodiments, the bicelle entity concentration is adapted to match the concentration of the DNA origami envelope. The DNA origami envelope advantageously avoids any destabilization of the bicelle entity.

In embodiments, the step x comprises diluting the bicelle entity in a buffer solution to have a substantially same concentration as the DNA origami envelope. In embodiments, the bicelle entity is immediately added to the provided DNA origami envelope for assembling. This advantageously avoids any destabilization. In further embodiments, the bicelle entity is diluted in step x and then immediately added to the provided DNA origami envelope for assembling.

In embodiments, the step x comprises removing the potential excess of bicelle entities compared to the concentration of the DNA origami envelope with an additional purification method. In embodiments, the additional purification method can be high-performance liquid chromatography (HPLC), ultracentrifugation, or dialysis.

In embodiments, the method further comprises a step y for forming the bicelle structure.

The step comprises providing a mixture of at least the first and second amphiphilic compounds and subjecting the mixture to repeated thermal cycling to induce phase separation between the first and second amphiphilic compounds.

In embodiments, the thermal cycling comprises alternating between a cooling step at a temperature below -50°C and a heating step at a temperature between 40°C and 80°C, with agitation between or during the heating step, wherein the thermal cycling is repeated at least 3 times.

In embodiments, the cooling step comprises freezing the mixture at a temperature below -150°C for a duration of 5 minutes or less, and the heating step comprises incubating the mixture in a water bath at a temperature between 55°C and 75°C for a duration between 2 and 10 minutes, followed by mechanical agitation for a duration between 10 seconds and 2 minutes.

In embodiments, the step comprises:
(a) providing a mixture of at least the first and second amphiphilic compounds, and
(b)
   (1) freezing the mixture in liquid nitrogen in one minute, directly followed by
   (2) a water bath at 65°C for 5 minutes and
   (3) vortexing for 30 seconds.
(c) repeating step (b) for at least 5 times.

The longer hydrophobic chain first amphiphilic compound is in the gel phase at room temperature while the shorter hydrophobic chain second amphiphilic compound is in the fluid phase at room temperature making them immiscible. Step x advantageously allows the separation of the second amphiphilic compound from the first amphiphilic compound, thus forming the bicelle structure.

In embodiments, the bicelle structure is formed in a separate container from the DNA origami envelope. This advantageously prevents the DNA origami envelope being damaged during the bicelle structure formation process.

In the seventh aspect, the invention relates to a method of manufacturing the hybrid pore device in the fifth aspect, comprising steps of:
i. Providing a biopore complex;
ii. Providing a solid-state pore with functionalization on a top surface for incorporating to the bicelle entity;
iii. Assembling the biopore complex and the solid-state pore.

In an embodiment of the seventh aspect, the invention relates to a method of manufacturing the hybrid pore device of the fifth aspect, comprising the steps of:
i. Providing a complex according to the third aspect;
ii. Providing a solid-state pore with functionalization on a top surface for incorporating to the bicelle entity of the complex;
iii. Assembling the complex and the solid-state pore.

### Brief description of the figures

Figure 1a is a top-view schematic illustration of a bicelle entity according to an exemplary embodiment.
Figure 1b is a top-view schematic illustration of a bicelle structure according to an exemplary embodiment.
Figure 1c is a side view schematic illustration of a bicelle structure according to an exemplary embodiment.
Figure 1d is a schematic illustration of an extension of the bicelle entity according to an exemplary embodiment.
Figure 2a is a top-view schematic illustration of a DNA origami envelope according to an exemplary embodiment.
Figure 2b is a side-view schematic illustration of a DNA origami envelope according to an exemplary embodiment. Figure 3a is a top-view schematic illustration of a complex according to an exemplary embodiment.
Figure 3b is a side-view schematic illustration of a complex according to an exemplary embodiment.
Figure 3c is a side-view schematic illustration of a complex according to a second exemplary embodiment.
Figure 4a is top and side view schematic illustration of a complex according to a reference experiment.
Figure 4b is top and side view schematic illustration of a DNA origami envelope according to a reference experiment.
Figure 5 is a side view schematic illustration of a complex with a biological pore according to an exemplary embodiment.
Figure 6 is a side view schematic illustration of a hybrid pore according to an exemplary embodiment.
Figure 7 is a picture of bicelle structures according to an exemplary embodiment.
Figure 8 is a picture of complex according to an exemplary embodiment.
Figure 9a is a picture of collapsed DNA origami envelope according to a reference experiment.
Figure 9b is a picture of DNA origami envelope according to a reference experiment.
Figure 10 is a picture of a DNA origami envelope and complex according to a reference experiment.
Figure 11 is pictures of experiments using detergents according to a reference experiment.
Figure 12a and 12b are pictures of bicelle structures fused together according to an experiment.
Figure 13a and 13b are pictures of complex according to an experiment.
Figure 14 is an AFM image of a DNA origami envelope with a DNA tail according to an experiment.

### Detailed description

The term "comprising", used in the claims, should not be interpreted as being restricted to the means listed thereafter; it does not exclude other elements or steps. It is thus to be interpreted as specifying the presence of the stated features, integers, steps, or components as referred to, but does not preclude the presence or addition of one or more other features, integers, steps or components, or groups thereof. The term "comprising" therefore covers the situation where only the stated features are present (and can therefore always be replaced by "consisting of" in order to restrict the scope to said stated features) and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

The term "a" shall be interpreted as a function word before a mass noun to denote a particular type or instance. It should not be interpreted as a function word before a singular noun referring one object.

Furthermore, "and/or" where used herein is to be taken as specific disclosure of each of the two specified features or components with or without the other. Thus, the term "and/or" as used in a phrase such as "A and/or B" herein is intended to include "A and B", "A or B", "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

An "amphiphilic compound" as herein disclosed refers to a chemical entity comprising both hydrophilic (water-attracting) and hydrophobic (water-repelling) regions within its molecular structure. This dual nature allows the compound to interact with both aqueous and lipophilic environments, facilitating the formation of micelles, bicelles, liposomes, or other self-assembled structures in a solution. The hydrophilic region typically contains polar or charged head groups, while the hydrophobic region consists of nonpolar hydrocarbon chains.

A "bicelle structure" as herein disclosed refers to an amphiphilic compound based structure comprising a mixture of long-chain (i.e., longer-chain) and short-chain (i.e., shorter chain) amphiphilic compounds. The long-chain amphiphilic compound forms a planar bilayer, while the shorter-chain amphiphilic compound stabilizes the edges of the bilayer by forming a rim, resulting in a discoidal or disk-like shape. In embodiments, "bicelle structure" refers to a lipid-based discoidal nanostructure comprising a mixture of long-chain and short-chain phospholipids.

"Longer-chain" or "Long-chain" and "shorter-chain" or "short-chain" amphiphilic compounds are defined relative to one another. Hence a "longer-chain" or "long-chain" amphiphilic compound is a compound having a longer hydrophobic chain than a "shorter-chain" or "short-chain" amphiphilic compound used in the bicelle structure. In example embodiments, a longer-chain amphiphilic compound as herein disclosed may refer to an amphiphilic compound having a hydrophobic chain length of 12 or more carbon atoms while a "shorter-chain amphiphilic compound" as herein disclosed may refer to an amphiphilic compound having a hydrophobic chain length of 10 or fewer carbon atoms, preferably 8 or fewer carbon atoms.

A "rim" of a bicelle structure as herein disclosed refers to the outer edge region where the short-chain amphiphilic compounds (e.g., phospholipids) stabilize the structure.

A "extension projecting from the rim" as herein disclosed refers to a molecular or structural feature that extends outward from the edge of the bicelle.

A "DNA origami envelope" as herein disclosed refers to a nanoscale structure formed by the precise folding of a long single-stranded DNA molecule into a predetermined shape, using shorter 'staple' strands to hold the structure together. This envelope can encapsulate and protect biologically active entities.

"Binding" as herein disclosed refers to a specific interaction between a ligand and a receptor, mediated by either covalent bonds or non-covalent forces such as hydrogen bonds, ionic bonds, van der Waals forces, or hydrophobic interactions. A "hydrophobic moiety" as herein disclosed refers to a nonpolar, water-insoluble segment of a molecule that exhibits an affinity for lipophilic environments. This segment typically consists of hydrocarbon chains or aromatic groups, which can interact with other hydrophobic entities through van der Waals forces and hydrophobic interactions.

A "hybrid pore" as herein disclosed refers to a composite structure that integrates the characteristics of a biopore and a solid-state pore. A "biopore" as herein disclosed refers to a biological structure with an opening in the structure that allows the passage of biological or chemical entities such as DNA molecules. A "solid-state nanopore" as herein disclosed refers to an aperture fabricated in an impermeable membrane. The membrane is typically thin and may have a thickness in the range of 0.3 nm to 1000 nm, such as between 1 nm and 100 nm. The membrane may be made of materials such as silicon nitride, silicon dioxide, or graphene.

A "lipid-handle" as herein disclosed refers to a hydrophobic moiety or lipid-like group that can be attached to a molecule, enabling it to interact with or embed itself into lipid membranes or hydrophobic environments.

### Examples

### Example 1: The complex comprising the bicelle entity (with both the first and second amphiphilic compound as described) and the DNA origami envelope

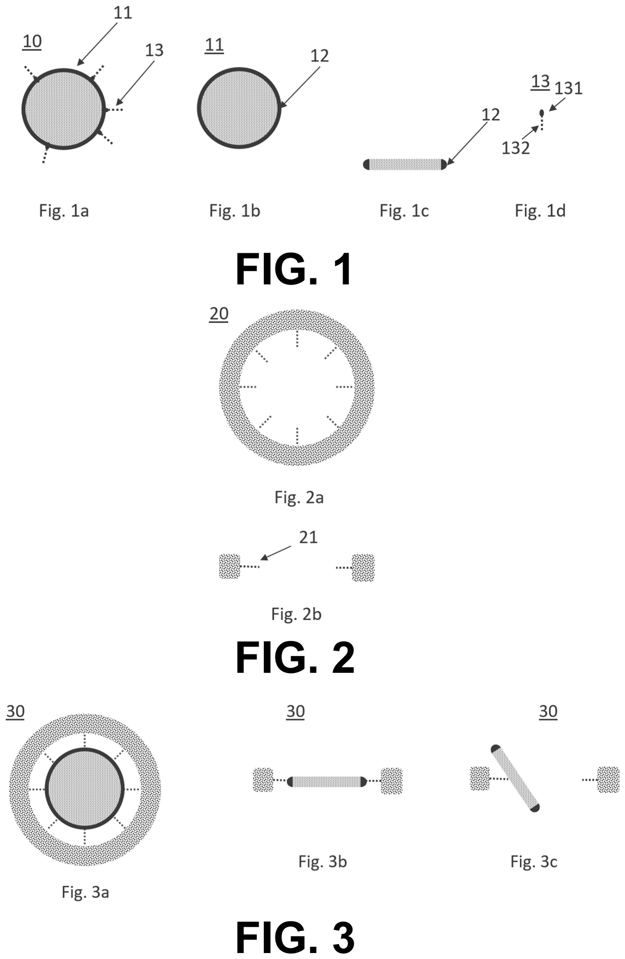

The bicelle structure 11, as shown in figure 1b and 1c, is formed via the described method. The bicelle structure 11 consists of 1,2-dimyristoyl-sn-glycero-3-phosphocholine (DMPC) as the first amphiphilic compound to form the bilayer central disc region, and 1,2-diheptanoyl-sn-glycero-3-phosphocholine (07:00 DHPC) to stabilize the bilayer DMPC disc by forming a surrounding rim 12. The ratio of long-chain lipid DMPC to the short-chain lipid DHPC is chosen to be 3, and more specifically, with lipid concentrations of 12 mM and 4 mM respectively. It must be noted that the DHPC concentration is above its critical micelle concentration (CMC) of approximately 1.4 mM. The method comprises:
(a) Providing the mixture of the first (DMPC) and second (DHPC) amphiphilic compounds with the specified concentrations, and
(b)
   (1) freezing the mixture in liquid nitrogen in one minute, directly followed by
   (2) a water bath at 65°C for 5 minutes and
   (3) vortexing for 30 seconds.
(c) repeating step (b) for 5 times.

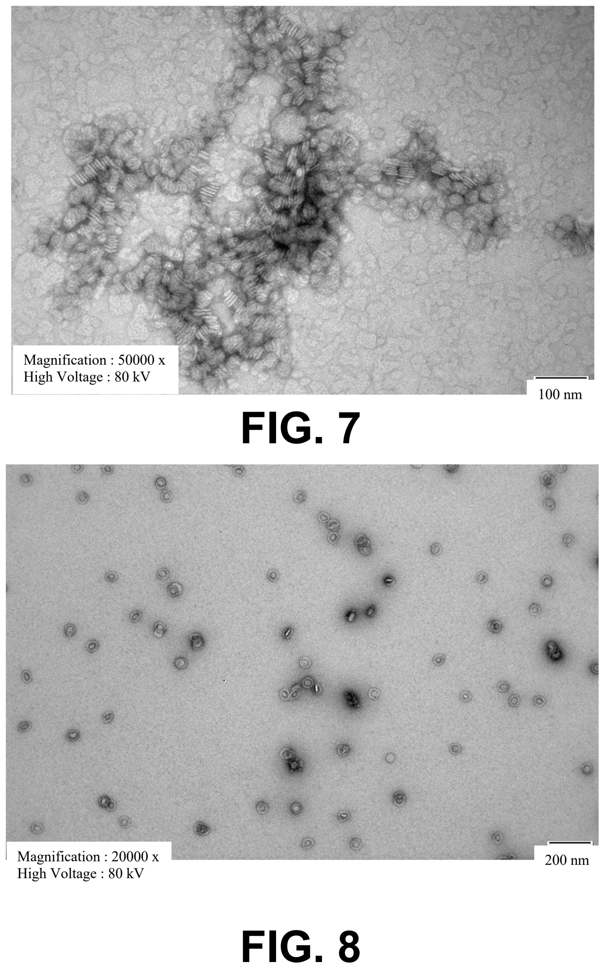

The obtained bicelle structures 11 have a discoidal structure with a diameter of approximately 26 ± 6 nm and a bilayer thickness of approximately 4.5 ± 0.8 nm (Figure 7).

The bicelle structures 11 are subsequently incubated with the extensions 13. The extensions, in this case ssDNA oligo, are bonded, or incorporated, into the bicelle structures 11 via a hydrophobic cholesterol moiety 131. The extension 13, as shown in Figure 1d, comprises a hydrophobic moiety 131, in this case cholesterol, anchored in the rim 12 of the bicelle structure 11, and a functional moiety 132, in this case a ssDNA oligo nucleic acid, projecting outward from the rim 12 for binding to the DNA origami envelope 20. Notably, this incubation is still carried out at a concentration for DHPC above its CMC to avoid the bicelle structure destabilization. The final bicelle entity 10, as shown in figure 1a, consists of a bicelle structure 11 functionalized with a random number of functional extensions (ssDNA) for assembling with the DNA origami envelope.

The DNA origami envelope 20 is in this case a ring structure with an outer diameter of 55 nm and an inner diameter of 35 nm. The DNA origami envelope 20 is shown in top view in Figure 2a and in side view in Figure 2b. The extensions 21, shown as ssDNA oligos extending inward from the inner surface of the DNA origami envelope 20, are configured for complementary binding to the extensions 13 of the bicelle entity 10. The structure also comprises 20 extensions configured for binding to at least one extension 13 of the bicelle entity 10. The extensions on the inner side of the DNA origami envelope are complementary ssDNA oligos able to hybridize, or bind, to the extensions 13 of the bicelle entity 10.

Subsequently, the bicelle entity 10 is diluted in buffer to have substantially the same concentration as the DNA origami envelope 20 and immediately added to the provided DNA origami envelope 20 for assembly. The hybridization of the complementary ssDNA oligos (the functional extensions) on both the bicelle entity 10 and the DNA origami envelope 20, ensures that the bicelle structure 11 is surrounded by the DNA origami envelope 20 (see Figure 8 for successful assembly). The resulting complex 30 is schematically shown in top view in Figure 3a and in side view in Figure 3b. As shown, the rim 12 of the bicelle structure 11 is entirely surrounded by the DNA origami envelope 20, with the extensions 13 of the bicelle entity 10 bonded to the extensions 21 of the DNA origami envelope 20 via complementary ssDNA hybridization. Figure 3c shows a side-view schematic of a complex 30 according to a second exemplary embodiment, in which the bicelle structure 11 is positioned at an angle relative to the plane of the DNA origami envelope 20. This illustrates that the binding between extensions 13 and the DNA origami envelope 20 accommodates variations in the orientation of the bicelle structure 11 within the DNA origami envelope 20. Notably, is that the final concentration of the short-chain DHPC lipid is now below the CMC. The highly charged DNA origami envelope 20 effectively stabilizes the bicelle structure 11 by limiting bicelle fusion through coulombic repulsion and steric hindrance (see example 3 to see the effect of bicelle fusion below the DHPC CMC). Interestingly, the incorporated bicelle structures 11 have an approximate size of 25 ± 6 nm, like the size of the bicelle structures measured before incorporation. Example 4 shows the effect of a different ratio in DMPC and DHPC lipids on the size of the incorporated bicelle structures 11 in the final assembly.

### Example 2 (reference): The complex comprising only the first amphiphilic compound and the DNA origami envelope

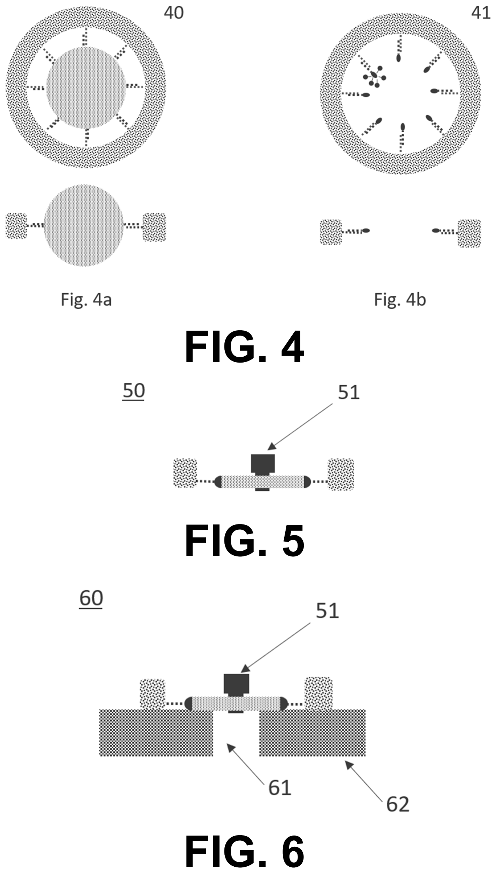

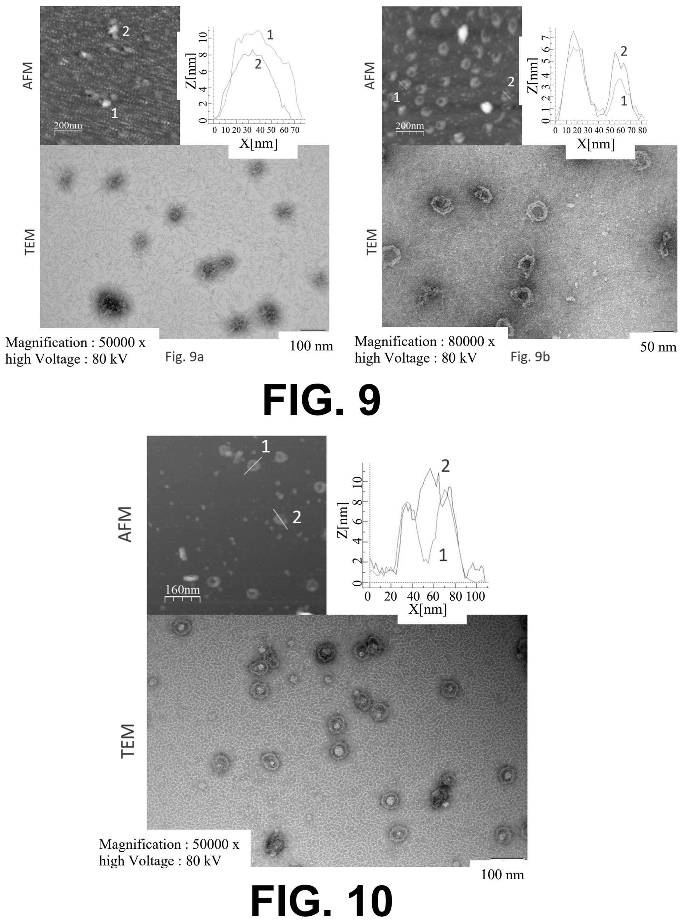

The DNA origami envelope is identical to the structure discussed in example 1. In this standard protocol, the extensions of the DNA origami envelope (ssDNA) are first hybridized to the complementary ssDNA extension connected to the hydrophobic cholesterol moiety able to connect the amphiphilic compound. This introduces the advantage of having a hydrophobic moiety connected to each extension on the inside of the DNA origami envelope. However, to avoid the collapse of the DNA structure, due to the hydrophobic moieties, detergent is added to ensure stabilization. Figure 4a shows a top and side view schematic of the complex 40 according to this reference experiment, in which only the first amphiphilic compound is incorporated into the DNA origami envelope 20 via the hydrophobic moieties connected to the extensions. Figure 4b shows the DNA origami envelope 41 according to this reference experiment, in which the hydrophobic moieties connected to the extensions are present on the inner surface but without an incorporated lipid structure, illustrating the collapsed or liposome-forming tendency in the absence of a proper bicelle structure. Figure 9a shows collapsed DNA origami envelopes with bound hydrophobic moieties that were not stabilized with detergent. Figure 9b shows the DNA origami envelope with bound hydrophobic moieties and stabilized with detergent.

Subsequently, a certain concentration of lipids, previously defined as the first amphiphilic compound, is added. The lipids are integrated into the DNA origami envelope via the bound hydrophobic moieties. Hereafter, the detergent is selectively removed via a detergent removal column.

However, this lipid incorporation strategy results in liposome insertion instead of a bilayer. The first amphiphilic compound specifically has a Critical Packing Parameter (CPP) of ~1. This is ideal to form a bilayer in a big structure but makes it impossible to form the rim of a discoidal bilayer, resulting in the formation of a curved liposome. In Figure 10, the height profile of cross-section 2 in the AFM figure shows that the incorporated lipids are larger than the surrounding DNA origami envelope, as compared to the empty DNA origami envelope from the height profile of cross-section 1. This suggests that a liposome is inserted instead of a thin bilayer.

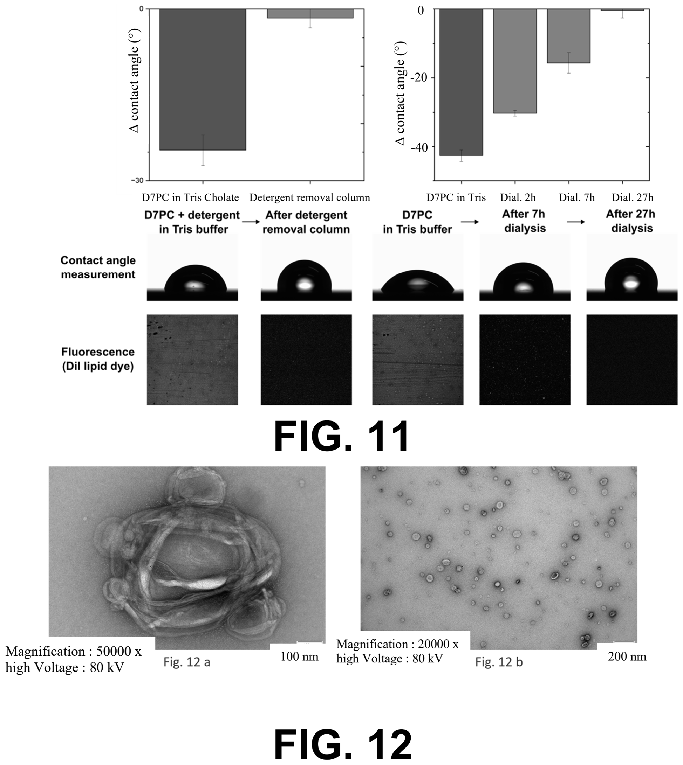

The novel bicelle incorporation protocol does not include the stabilization with detergent. This is because the detergent cannot be selectively removed from the second amphiphilic compound, which closely resembles a detergent itself (Figure 11 shows that a detergent removal column and dialysis remove both the detergent, in this case sodium cholate, and the second amphiphilic compound, in this case 07:00 DHPC). The usage of a detergent must thus be avoided when incorporating a bicelle structure.

### Example 3: The bicelle entity (with both the first and second amphiphilic compound as described) but without the DNA origami envelope

The bicelle structure 11, as shown in figure 1b and 1c, is formed via the described method. The bicelle structure 11 consists of 1,2-dimyristoyl-sn-glycero-3-phosphocholine (DMPC) as the first amphiphilic compound to form the bilayer central disc region, and 1,2-diheptanoyl-sn-glycero-3-phosphocholine (07:00 DHPC) to stabilize the bilayer DMPC disc by forming a surrounding rim 12. The ratio of long-chain lipid DMPC to the short-chain lipid DHPC is chosen to be 3, and more specifically, with lipid concentrations of 12 mM and 4 mM respectively. It must be noted that the DHPC concentration is above its critical micelle concentration (CMC) of approximately 1.4 mM.

When the bicelle structure 11 is diluted to match the DNA origami envelope 20 concentration (as described in Example 1) but not immediately added to the DNA origami envelope for stabilization, the structures become unstable. Without the stabilizing effect of the DNA origami envelope, the bicelle structures 11 rapidly fuse together below the critical micelle concentration (CMC) and form large multilamellar vesicles (see Figure 12a).

Similarly, when the complete bicelle entity 10, including the extensions 13, is diluted without immediate addition to the DNA origami envelope, it also undergoes bicelle fusion and loses its discoidal bilayer structure (Figure 12b).

### Example 4: The complex comprising the bicelle entity (with both the first and second amphiphilic compound as described) but of different sizes and the DNA origami envelope

The bicelle structure 11, as shown in figure 1b and 1c, is formed via the described method. The bicelle structure 11 consists of 1,2-dimyristoyl-sn-glycero-3-phosphocholine (DMPC) as the first amphiphilic compound to form the bilayer central disc region, and 1,2-diheptanoyl-sn-glycero-3-phosphocholine (07:00 DHPC) to stabilize the bilayer DMPC disc by forming a surrounding rim 12. The ratio of long-chain lipid DMPC to the short-chain lipid DHPC is now chosen to differ from the previously selected ratio of 3, more specifically 1 and 2. This results in DMPC lipid concentrations of respectively 4 and 8 mM, while the DHPC concentration remains 4 mM. It must be noted that the DHPC concentration is again above its critical micelle concentration (CMC) of approximately 1.4 mM.

A change in the ratio of the first and second amphiphilic compounds results in the formation of bicelle structures 11 of a different size. These bicelle structures 11 are subsequently also mixed with the extension 13 containing a hydrophobic moiety, in this case a cholesterol moiety, and a functional moiety 132, in this case a ssDNA oligo 132 for connection to the DNA origami envelope 20.

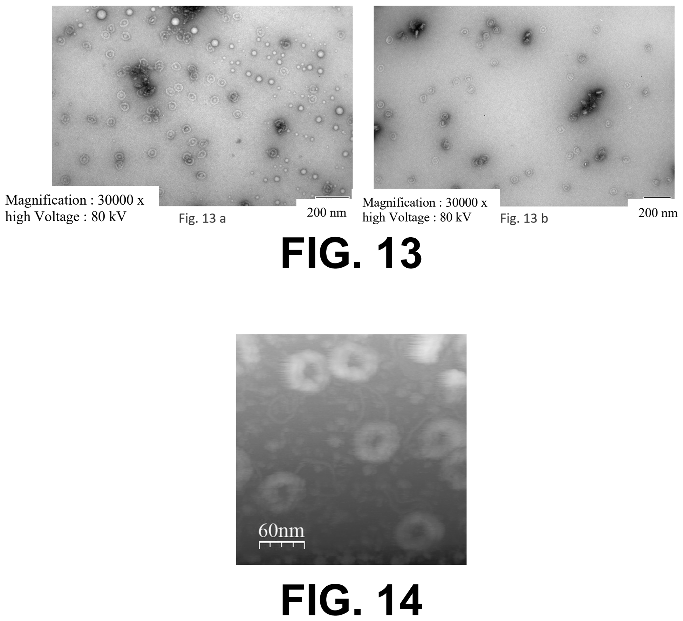

Hereafter, both bicelle entities 10, with different lipid ratios, are assembled into the DNA origami envelope 20 for stabilization. As can be seen in Figure 13a and 13b, the incorporated bicelle entities 10 vary in size depending on their original lipid ratio. For the lipid ratio of 1, this results in an incorporated bicelle entity 10 with a size of approximately 17 ± 3 nm. For the lipid ratio of 2, this results in an incorporated bicelle entity 10 with a size of approximately 21 ± 4 nm.

### Example 5: The complex comprising the bicelle structure and the DNA origami envelope is used to incorporate a membrane protein, like a biopore

To study membrane proteins via single-molecule sensing techniques, e.g. EM imaging and NMR, in their native environment, protein nanodiscs are often used. These protein nanodiscs often use the 1,2-dimyristoyl-sn-glycero-3-phosphocholine (DMPC) lipids but are also often too small (~10 nm) for large membrane proteins. This limits the available area that can be replaced by a membrane protein and its space to freely move in the membrane.

The complex presented here with a bicelle structure based on 1,2-dimyristoyl-sn-glycero-3-phosphocholine (DMPC) and the DNA origami envelope can provide a larger native environment for the study of membrane proteins.

Cytolysin A (ClyA) is an example of a relatively large biological nanopore 51 that can be incorporated into the complex 30. The complex 50 with the incorporated biological nanopore 51 is schematically shown in side view in Figure 5. The biological nanopore 51 is embedded in the bilayer of the bicelle structure 11, with the DNA origami envelope 20 surrounding the rim 12, thereby positioning the biological nanopore 51 in a stable, native-like membrane environment.

### Example 6: The complex comprising the bicelle structure and the DNA origami envelope is used as an interface between a biopore and a solid-state nanopore resulting in a hybrid nanopore complex

The variability in size of solid-state nanopores 61 can benefit from the atomically-precise dimensions of biological nanopores 51.

The complex 30 comprising the bicelle structure 11 and the DNA origami envelope 20 is then used as an interface between the biopore 51, i.e. the biological pore and the solid-state nanopore 61 resulting in a hybrid nanopore complex 50.

The DNA origami envelope 20 in this example has a further feature, namely a specifically designed dsDNA tail, or handle, to allow for the positioning of the bicelle complex, including the biopore 51, at the solid-state nanopore 61. The DNA tail is pulled towards the solid-state nanopore 61 by the electrophoretic force, and hence the complex is aligned and forms a hybrid nanopore complex 60. The hybrid pore device 60 is schematically shown in side view in Figure 6. The complex 50 comprising the bicelle structure 11, the DNA origami envelope 20, and the biological nanopore 51 is assembled on a substrate 62 comprising a solid-state nanopore 61. The biological nanopore 51 and the solid-state nanopore 61 are aligned to allow the passage of an analyte entity through both pores. An AFM image showing the DNA tail on the DNA origami envelope 20 is shown in Figure 14.

## Claims

1. A bicelle entity (10) comprising:
A bicelle structure (11) comprising a rim (12), a first and a second amphiphilic compounds, wherein the first amphiphilic compound has a longer hydrophobic chain than the second amphiphilic compound;
At least one extension (13) projecting from the rim (12), wherein the at least one extension (13) is configured for binding to a DNA origami envelope (20) or an extension (21) of the DNA origami envelope (20).

2. A bicelle entity (10) according to claim 1, wherein the at least one extension (13) comprises a hydrophobic moiety (131) and is bonded to the bicelle structure (11) via said hydrophobic moiety (131), wherein the hydrophobic moiety (131) is optionally selected from the list consisting of cholesterol, porphyrin, tocopherol, and a lipid-handle.

3. A bicelle entity (10) according to any of the preceding claims, wherein the extension (13) comprises a functional moiety (132) for binding to the DNA origami envelope or the extension of the DNA origami envelope, wherein the functional moiety (132) is optionally a ssDNA oligo nucleic acid.

4. A bicelle entity (10) according to any of the preceding claims, wherein a Critical Packing Parameter of the first amphiphilic compound is in the range between 0.5 to 1.1.

5. A bicelle entity (10) according to any of the preceding claims, wherein a Critical Packing Parameter of the second amphiphilic compound is below or equal to 0.5.

6. A bicelle entity (10) according to any of the preceding claims wherein the bicelle entity (10) is configured so that the rim (12) of the bicelle structure (11) can be surrounded by the DNA origami envelope (20).

7. A DNA origami envelope (20) configured for binding to the at least one extension (13) of the bicelle entity (10) of any of the claims 1 to 6.

8. A DNA origami envelope (20) according to claim 7, wherein the DNA origami envelope (20) is configured for binding to the at least one extension (13) such that the rim (12) of the bicelle structure (11) is entirely surrounded by the DNA origami envelope (20).

9. A complex (30) comprising a bicelle structure (11) binding to a DNA origami envelope or an extension of the DNA origami envelope (20).

10. A complex (30) according to claim 9, further comprising a protein incorporated into the bicelle structure.

11. A complex (30, 50) according to claim 10, wherein the protein is a biological pore (51).

12. A kit of parts, comprising:
- A bicelle entity (10) according to any of the claims 1 to 9 and
- A DNA origami envelope (20) configured for binding to the at least one extension of the bicelle entity.

13. A hybrid pore device (60) comprising a complex (50) according to claim 11.

14. A method of manufacturing the complex (30,50) according to any of the claims 9 to 11, comprising steps of:
a. Providing a bicelle entity (10) according to any of the claims 1 to 6;
b. Providing a DNA origami envelope (20) according to any of the claims 7 to 8;
c. Assembling the bicelle entity (10) and the DNA origami envelope (20).

15. A method of manufacturing the hybrid pore device (60) according to any of the claims 20 to 23, comprising steps of:
i. Providing a complex (50) according to claim 18;
ii. Providing a solid state pore (61);
iii. Assembling the complex (50) and the solid state pore (61).
